(19) Europäisches Patentamt
European Patent Office
Office européen des brevets

(11) EP 2 184 991 B1

(12) # EUROPEAN PATENT SPECIFICATION

(45) Date of publication and mention of the grant of the patent:
**02.09.2015 Bulletin 2015/36**

(21) Application number: **07803625.8**

(22) Date of filing: **05.10.2007**

(51) Int Cl.:
*A01N 43/90* (2006.01) *A01N 47/44* (2006.01)
*A01N 25/30* (2006.01) *A01P 1/00* (2006.01)
*A01P 3/00* (2006.01) *A23C 19/11* (2006.01)
*A23L 3/3463* (2006.01)

(86) International application number:
**PCT/EP2007/060598**

(87) International publication number:
**WO 2009/033508 (19.03.2009 Gazette 2009/12)**

(54) **COMBINATIONS OF POLYENE FUNGICIDE WITH CATIONIC SURFACTANTS**

KOMBINATIONEN VON POLYEN-FUNGIZID MIT KATIONISCHEN TENSIDEN

ASSOCIATIONS D'UN FUNGICIDE DE TYPE POLYÈNE AVEC DES TENSIOACTIFS CATIONIQUES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **13.09.2007 ES 200702132**

(43) Date of publication of application:
**19.05.2010 Bulletin 2010/20**

(73) Proprietor: **Laboratorios Miret, S.A.**
**08228 Les Fonts de Terrassa (ES)**

(72) Inventors:
- **ROCABAYERA BONVILA, Xavier**
  **E-08184 Palau-solita (ES)**
- **FIGUERAS ROCA, Sergi**
  **E-08005 Barcelona (ES)**
- **SEGRET PONS, Roger**
  **E-08023 Barcelona (ES)**
- **PIERA EROLES, Eva**
  **E-08025 Barcelona (ES)**

(74) Representative: **Gille Hrabal**
**Brucknerstrasse 20**
**40593 Düsseldorf (DE)**

(56) References cited:
**EP-A- 0 513 922**
**WO-A-2004/082407**
**WO-A-2006/084553**
**WO-A-2007/038627**
**WO-A-2007/112379**
**WO-A-2008/104417**
**WO-A-2009/031041**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 184 991 B1

## Description

### *Field of the Invention*

The present invention refers to combinations of the polyene fungicide natamycin with the cationic surfactant LAE with antimicrobial properties to improve the preservative activity of both compounds against microorganisms responsible for the spoilage of food and of cosmetic and pharmaceutical products.

### *Background Art*

In the art it is known that food products are regularly susceptible to act as culture medium for microorganisms and this constitutes a possible risk to human health. As food-borne disease remains a real problem in both developed and developing countries, causing great human suffering and significant economic loss, food products require good protection against microbial contamination.

The polyene fungicide natamycin (CAS No. 7684-93-8), also known as pimaricin, is a naturally occurring antimicrobial agent produced by the bacterium *Streptomyces natalensis.* This compound is effective against yeasts and moulds, but it has been reported that it is ineffective against bacteria. It is used in the food industry as a preservative to inhibit fungal growth (approved in the European community under E-235; in the USA it is regulated by 21 CFR 172.155). For more than 20 years, natamycin has been used to prevent growth of mold on cheese and sausages. Besides, it is also used in other foodstuffs that are subject to microbial spoilage such as dressings, sauces, marinades, condiments, spreads, margarine and dairy based foods.

The lack of solubility of polyene fungicide in various solvents (aqueous as well as organic) considerably restricts its use. Due to the instability of polyene fungicide in solution, it is known to be impossible to obtain an aqueous solution of polyene fungicide (EP 0 678 241 B1). The solubility of natamycin in water is very low and in the order of 0.005-0.010 % (w/w).

For this reason, natamycin has been applied to foodstuff in different ways, for example, in a dry form, in aqueous suspensions, by mixing natamycin with water-miscible solvents to reach an stable aqueous solution, etc. Use of the dry form implies the difficulty to obtain a homogeneous distribution. Use of the aqueous suspension implies the formation of precipitates due to the low stability of natamycin. Several attempts have been made to solubilise polyene fungicides such as natamycin with the purpose to improve their efficacy.

EP 0 670 676 B1 concerns a novel type of natamycin preparation that implies a modified form of natamycin by means of contacting the natamycin with methanol to convert the natamycin in a solvated form and then removing the methanol form the natamcyin to form $\delta$-natamycin. The inventors surprisingly found that $\delta$-natamycin has an improved dissolution behaviour.

EP 0 678 241 B1 discloses an aqueous suspension of natamycin that contains a thickening agent and has a pH between 3-6. Applying a suitable pH range along with a thickening agent, the inventors obtained an aqueous suspension of natamycin chemically and microbially stable for more than 14 days and physically stable for several hours.

US 5,597,598 relates to a composition comprising a polyene antifungal agent, like natamycin, an acidic antifungal compound and an additional acid compound. This composition is useful to prevent the growth of mold which are more tolerant towards polyene fungicides. This composition can be incorporated into a coating emulsion or in a liquid where the food and agricultural products wanted to treat can be brushed with the coating emulsion or immersed in the liquid.

US 6,146,675 concerns a novel antimicrobial composition comprising natamycin and an oxygen scavenger or antioxidant, and/or chelating agent. Water hardness control is used to sustain the natamycin activity by preventing its degradation. WO2005097063 relates to a specific process and formulation for producing natamycin tablets to facilitate their preparation and use in the food and feed industry and to ensure that such tablets rapidly and fully disintegrate when added to a liquid vehicle.

On the other hand, cationic surfactants are known as preservatives used in food, cosmetic and pharmaceutical industry. Cationic surfactants have turned out to be highly effective against microbial proliferation and at the same time safe for intake in humans and mammals in general. For all of this, cationic surfactants are an attractive tool in the industry.

It has been demonstrated that cationic surfactants according to formula (1) derived from the condensation of fatty acids and esterified dibasic amino acids are highly effective protective substances against microorganisms.

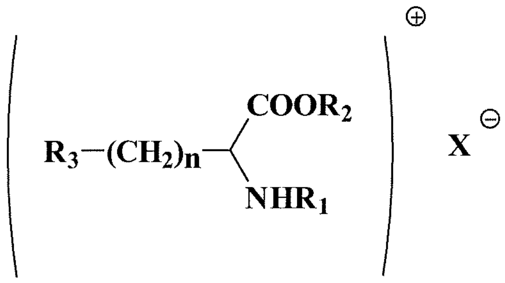

(1)

where:

$X^-$ is a counter ion derived from an inorganic or organic acid, preferably $Br^-$, $Cl^-$, or $HSO_4^-$
$R_1$: is a straight alkyl chain of a saturated fatty acid or a hydroxy acid having 8 to 14 carbon atoms linked to the α-amino group via an amide bond,
$R_2$: is a straight or branched alkyl chain from 1 to 18 carbon atoms or an aromatic group and
$R_3$: is:

**$-NH_3$**

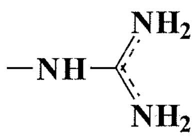

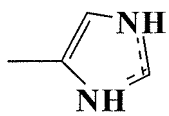

In particular the ethyl ester of the lauramide of the arginine monohydrochloride, hereafter referred to as LAE (CAS No. 60372-77-2), is now well-known for its use as an antimicrobial agent. In practical use LAE turned out to be well tolerated and to display a very low toxicity to human beings. LAE has the chemical structure of formula (2) displayed hereafter.

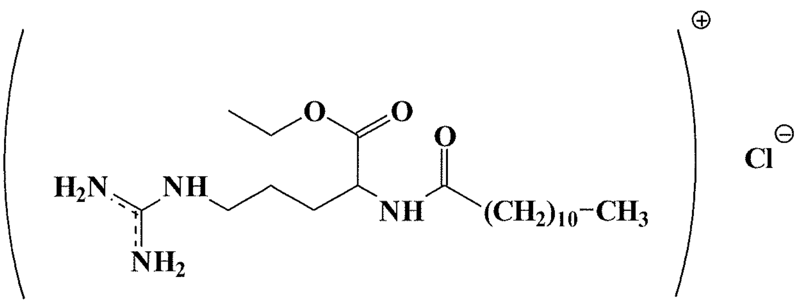

(2)

The compound LAE is remarkable for its activity against different micro-organisms, like bacteria, moulds and yeasts which can be present in food products (WO 03/034842) and also in cosmetic formulations and preparations (WO 03/013453, WO 03/013454 and WO 03/043593). The product is outstanding for its innocuity to humans.
The general preparation of the cationic surfactants is described in Spanish patent ES 512643 and international patent applications WO 96/21642, WO 01/94292 and WO 03/064669.
Interactions between the cationic surfactants and other molecules are known. A combination of the cationic surfactants with anionic hydrocolloids is described in WO 03/094638, this combination leads to the generation of solid products containing approximately stoichiometric amounts of the cationic surfactant and the anionic hydrocolloid. A

further combination of the cationic surfactants is described in (US 7,074,447 and EP 1 450 608 B1), this combination relating to potassium sorbate, calcium sorbate or sorbic acid, which turned out to be highly effective in food preservation. The preservative systems described in (US 7,074,447 and EP 1 450 608 B1) are characterised by their synergistic activity.

It has also been found that the antimicrobial activity of the combinations of LAE and the other compounds defined by the above formula (1) with most of the common ionic and non-ionic preservatives used to protect food products and also cosmetic formulations and preparations is higher than the activity displayed by each of the components when used alone at the same dosage. There has been observed synergism when the amounts of the compounds of formula (1) and the other antimicrobial are reduced. Thus, the adverse toxic effects and/or irritation and/or allergy displayed by the combinations of the preservatives have also been reduced.

LAE, also known as lauric arginate, is manufactured by Laboratories Miret, S.A. (LAMIRSA, Spain). Lauric arginate is listed by the FDA (Food and Drug Administration) as being a GRAS substance (Generally Recognized As Safe) under GRN 000164. The USDA (United States Department of Agriculture) has approved its use in meat and poultry products (FSIS Directive 7120.1).

In WO 2006/084553 A1 and WO 2004/082407 A1 food compositions are described in which LAE and natamycin are present at the same time.

In WO 2007/112379 A1 combinations of LAE with an antibiotic selected from β-lactam antibiotics, polypeptides and quinolones are described.

There is a need to provide a stable aqueous solution of natamycin in order to improve its application in a homogeneous distribution for the treatment of food products, of devices to prepare food products, of medical devices or cosmetic and medical products where the growth of microorganisms is common.

There is also a need for a process for the preparation of a stable aqueous solution of natamycin which is effective and easy and allows the preparation of solutions of any suitable concentration.

### *Summary of the Invention*

The invention provides a solid composition consisting of the polyene fungicide natamycin and LAE (represented as formula (2) above).

The invention also provides a method for preparing a dispersion of natamycin by the dispersion of the solid composition according to the invention in a suitable liquid medium.

The invention further provides a dispersion of natamycin prepared by the method of the invention.

And the invention provides a method for preparing an aqueous solution of natamycin by diluting the dispersion with water.

This invention solves the problems of the generally low solubility of natamycin by means of providing an aqueous composition constituted by the cationic surfactant of formula (2) which encapsulates the polyene fungicide natamycin. This composition surprisingly improves the activity of natamycin because it increases the bioavailability of natamycin in the aqueous phase which is the medium where microorganisms grow. Encapsulating natamycin in a cationic surfactant is a way to avoid that it precipitates. At the same time it improves its availability in the matrix where the aqueous solution is applied. Unexpectedly, once the cationic surfactant encapsulates natamycin in the aqueous solution, the polyene fungicide is released to the matrix, where the aqueous solution is applied, in a dose level which is always solubilised, stable and much more effective.

Polyene fungicides are in general effective against yeasts and moulds and ineffective against bacteria. Cationic surfactants due to their cationic properties are easily linked to the membranes of bacteria. The inventors unexpectedly observe that after encapsulating natamycin in LAE in an aqueous solution a synergistic antimicrobial effect is observed.

Encapsulating natamycin in LAE provides an aqueous solution which is stable and effective against yeasts, moulds and bacteria and it is surprisingly observed a synergistic effect.

### *Description of preferred embodiments*

The present inventors have found, that it is possible to provide a solid composition consisting essentially of the polyene fungicide natamycin and the cationic surfactant of the above formula (2).

The cationic surfactant which is used in the present invention is the ethyl ester of lauric arginate of above formula (2) (hereafter referred to as LAE).

The second component of the solid composition is the polyene fungicide natamycin.

The solid composition may contain further components such as sugar, salt, anticakings, antioxidants, chelating agents, surfactant agents and thickening agents. Such further components may function to stabilize the solid composition itself, for instance the presence of an antioxidant may help to prolong the storage stability of the solid composition. The further components may also function to improve the dispersed form of the solid dispersion or its more diluted solution in water, which dispersed form and solution are the preferred processed forms of the solid composition which will be

discussed hereafter.

The presence of the further components may help to prepare the dispersed form of the polyene fungicide by simple dispersion in a solvent, without the need to add any further supplement.

In the same manner, the presence of the further components may help to prepare the aqueous solution of the polyene fungicide, without the need to provide any particular solution in water for the preparation of the solution, just water itself may be sufficient to achieve the wanted effect.

The solid composition essentially consists of natamycin and LAE.

The amount of the two components natamyin and LAE may vary depending on the intended use. It may be appropriate to provide a solid composition with a relatively increased amount of natamycin or a solid composition with a relatively increased amount of LAE.

The preparation of the dispersion of natamycin which will be described hereafter involves the simple dispersing step in a suitable solvent. The preparation of the solution of natamycin involves the further dilution of the dispersion with water or the dissolution of the solid composition in water. In either of these preparations there is no intention of a further addition of the polyene fungicide or the cationic surfactant. So, the final relative amounts of the natamycin and LAE in the dispersion and the solution are already determined by their relative presence in the initially provided solid composition. In the later treatment of the solid composition through the method of dispersion or solution further natamycin or LAE could be added in practice, but this further addition would necessarily complicate the preparation method and it does not constitute the preferred method of the invention.

The mixture of LAE and natamycin consists of 2.0 - 99.9 % by weight of LAE and 0.1 - 98.0 % by weight of natamycin, the sum of the two being 100%.

The preferred mixtures of the LAE and the natamycin cover a wide range of mixtures because each range has a specific effect.

For example, the mixture constituted by 99.0% of LAE and 1.0% of natamycin is preferred to be used in those foodstuffs where the initial presence of microorganisms like yeast and moulds is in a low concentration. The mixture allows that natamycin has a constant effect against the microorganisms.

Another preferred mixture is intended for curing food products whereby the curing process is short but the initial concentration of yeast and mould is very high. The preferred mixture is constituted by 2% of LAE and 98% of natamycin. Thanks to this mixture the high concentration of yeast and mould at the beginning of the curing process is reduced considerably, avoiding, in this sense, the initial attack of these microorganisms in the food.

There is another preferred mixture which is characterized by a relationship 1:1 between LAE and natamycin. This mixture is preferred in those products which are subjected to a short curing process whereby the initial concentration of microorganisms is low or to a long curing process when the initial concentration of microorganisms is high.

The composition may further contain an amount of further ingredients, as discussed before. The amount of the further ingredients depends on the intended final use of the solid dispersion. A usual amount of the further ingredients may be in the range of 0 to 5 parts by weight to a total of 100 parts by weight of the mixture of natamycin and LAE

The solid composition can be prepared using conventional methods for preparation. A convenient method is to provide the two components in a solid mixer and to mix the components in the mixer for a sufficient time until a homogenous mixture is obtained. If further components are intended to be present, these may be added together with the components LAE and natamycin or they may be added after the homogeneous mixture has been obtained. The composition which is finally obtained is a powdery substance.

The solid composition can be stored for a considerable duration of time without need to take specific measures of caution. It is generally recommended to store the solid compositions under conditions of low humidity and a temperature not exceeding 20°C, but conditions outside the preferred range allow storage for a long duration as well.

The solid composition of the invention is particularly suitable to be used for the preparation of a dispersion of natamycin in any suitable liquid. The dispersion is meant to relate to a liquid containing particles in a size of 50 $\mu$m to 10 nm. The dispersion may have a certain turbidity which may be as strong as to give it a milky look. The final look of the dispersion is determined by the size and the concentration of the particles in the dispersing liquid. The dispersion may be considered as a concentrated preparation of natamycin the concentration being well above the range in which natamycin would be used as an antimicrobial agent in food products.

The liquid basis of the dispersion may be any liquid which is suitable for use in the preparation of food. Such liquids are water, propylene glycol, ethanol, or glycerine. Mixtures of these liquids are possible as well.

Water may refer to tap water, demineralised water, distilled water, or solutions of any suitable salt in water.

Particularly preferred is tap water.

The dispersed phase in the dispersion is essentially consisting of LAE and natamycin. It may also contain the further ingredients, if these are present. Alternatively the further ingredients may have dissolved in the liquid which is used for the preparation of the dispersion.

The preparation of the dispersion is particularly easy. The liquid which is selected for the step of dispersion is added to the solid composition or the solid composition is added to the liquid. The mixture is then stirred for a time which

is sufficient to obtain the wanted dispersion. No remaining particulate material may be observed. The wanted degree of dispersion may be controlled by the conventional methods such as turbidimetry.

A dispersion of natamycin prepared in this manner may display a concentration of this polyene fungicide of between 100 ppm and 10,000 ppm, optionally comprising ingredients such as sugar, salt, anticakings, antioxidants, chelating agents, surfactants and thickening agents.

The concentration of natamycin in the dispersion may preferably vary between 100 ppm and 5000 ppm. The corresponding concentration of LAE in the dispersion depends on the relative presence of the two components in the solid dispersion.

It is one of the surprising aspects of the present invention, that the dispersion of natamycin displays a high degree of stability. The dispersion may be stored for more than 12 months without any observation of the occurrence of precipitation and without loss of any of its original optical appearance, a dispersion which is initially slightly turbid will remain to be so after 12 months. The dispersion should be stored in closed vessels but otherwise there is no need for a special treatment during storage.

It is the special advantage of the inventive dispersion of the polyene fungicide natamycin that it is the highly suitable basis for the subsequent preparation for a solution of the polyene fungicide in water. The solution of the polyene fungicide in water is the preparation which constitutes the most ideal preparation for the treatment of food products. The typical use of natamycin is the treatment of cheese. It is very appropriate to prepare a suitable bath in which the cheeses are allowed to float for a specific period until the wanted effect has set in. The invention therefore provides the method of preparing a solution of natamycin by diluting the dispersion of natamycin which is described above with water. This preparation is particularly easy and comfortable. The dispersion according to the invention is added to the vessel containing water, or alternatively water is added to a vessel already containing the dispersion. The mixture must be mixed until a homogeneous solution has been obtained. The duration of the stirring step is not particularly long, stirring with a conventional stirring device for 10 to 20 minutes is usually sufficient to achieve the homogeneous solution.

The solution which is obtained is clear and usually no precipitate may be observed and no turbidity is accepted. However, in some occasions a certain degree of turbidity or a slight precipitate may appear, which in all cases is completely acceptable as it is considerably lower than in a sample with only natamycin.

It is also possible to prepare the solution by processing the solid composition in a corresponding volume of water immediately and accordingly to omit the stage of the separate storage as a dispersion. This is usually less convenient than the preparation of the solution on the basis of the dispersion, extensive stirring of considerable duration may be needed to achieve the wanted result and the control of the final result is less easy compared with the dilution of the dispersion.

And of course, it is also possible to prepare the aqueous solution from the components natamycin and LAE themselves, adding these components to a suitable amount of water and stirring until homogeneity. In this manner of preparation it is preferred than LAE is initially added to the liquid phase and the polyene fungicide is added in the second step. The manner of preparation is less convenient and it involves considerable stirring energy, but it is possible and may lead to the same final result.

The stability of the solution is high. Preparation of a solution by the dilution of the dispersion has shown that the solution is stable for 6 months. Therefore the solution may be prepared immediately, but usually the intermediate preparation of the more concentrated dispersion may overcome a lot of practical storage problems.

The final concentration of the components natamycin and the cationic surfactant LAE in the aqueous solution is determined by the concentration of these components in the preparation from which the aqueous solution is prepared.

The aqueous solution may contain natamycin in a concentration of 100 to 10,000 ppm in combination with LAE in an amount of 200 to 100,000 ppm and optionally comprising ingredients such as sugar, salt, anticakings, antioxidants, chelating agents, surfactants and thickening agents.

According to a preferred embodiment the aqueous solution contains the two components in a ratio by weight of natamycin to LAE of 1:20 to 1:200, preferably 1:50 to 1:100.

According to a further preferred embodiment the aqueous solution according to the present invention contains natamycin at a concentration of 0.001 to 0.15 % (w/w), preferably of 0.01 to 0.10 % (w/w).

The mixture of natamycin (commercially available) with a cationic surfactant like LAE surprisingly solubilised the natamycin in an aqueous solution. According to the dose level between natamycin and LAE, the total solubility of natamycin was observed. The aqueous solution obtained had a low viscosity as there is no need to add in the solution gums or other thickening substances to stabilize the natamycin. This low viscosity improves the application process of the solution in the product desired to be treated.

The following table shows the relationship between natamycin and LAE to reach the solubility of natamycin in an aqueous solution:

| Natamycin (ppm) | LAE (ppm) | Relationship | Status Aqueous Solution |
|---|---|---|---|
| 100 | 3.000 | 1:30 | Slight Precipitate |
| 100 | 5.000 | 1:50 | Solubility |
| 100 | 50.000 | 1:500 | Solubility |
| 250 | 10.000 | 1:40 | Slight Precipitate |
| 250 | 20.000 | 1:80 | Solubility |
| 500 | 30.000 | 1:60 | Slight Precipitate |
| 500 | 45.000 | 1:90 | Solubility |
| 1.000 | 100.000 | 1:100 | Solubility |

The results show that natamycin was better solubilised when it was mixed with the highest doses of LAE. The solubility of natamycin is depending on the concentration. A high concentration of natamycin implies a high concentration of LAE to solubilise the natamycin.

The inventors have observed that 10% of LAE in water allowed to solubilise more than 1000 ppm of natamycin.

It is a further surprising effect observed in the present invention, that the combination of natamycin with LAE displays a biological effect which is better than might be expected on the basis of the knowledge of the effects displayed by the single components.

The solution of the combination of natamycin with LAE is suitable for the treatment of food products. All kind of food products which are treated with natamycin may be treated with the combination of the present invention.

The food products which may be treated are products such as cheese, meat products, in particular cold meat products and bakery products. The application of the aqueous liquid is performed by immersion or spraying, the choice of the application method depending on the kind of product to be treated.

Further products to be treated may be drinking fluids. In this case the application is completed by adding the inventive composition to beverages such as orange juice.

Other applications may relate to products which are not intended to be consumed. For instance cosmetic products may be treated by spraying with the aqueous solution or by adding the inventive composition directly to the product.

And finally, each of the products of the invention, the solid composition, the dispersion and the aqueous solution, may be used for the preparation of pharmaceutical preparations for the treatment of human beings or animals, the treatment being directed to any kind of disease connected to infections with bacteria or fungi.

EXAMPLES.

Example 1. 198 g of LAE (producer LAMIRSA) is provided into a solid mixer (producer: ORTOALRESA). An amount of 2 g natamycin (producer: Sigma) is added. The mixture of the two compounds is mixed in the solid mixer at 60 rpm for 30 minutes.

Example 2. 196 g of natamycin (producer Sigma) is provided into a solid mixer (producer: ORTOALRESA). An amount of 4 g LAE (producer: LAMIRSA) is added. The mixture of the two compounds is mixed in the solid mixer at 60 rpm for 30 minutes.

Example 3. 100 g of LAE (producer LAMIRSA) is provided into a solid mixer (producer: ORTOALRESA). An amount of 100 g natamycin (producer: Sigma) is added. The mixture of the two compounds is mixed in the solid mixer at 60 rpm for 30 minutes.

Example 4. 400 g of a mixture according to example 1 is provided in a container. 600 ml tap water is added to the container.

The mixture is stirred for 30 minutes with a stirrer (produced by HEIDOLPH) at room temperature.

At the end of the stirring procedure a non-clear liquid of white colour was obtained. The concentration of LAE in the dispersion was 39.6 % by weight (w/w).

The concentration of natamycin in the dispersion was 0.4% by weight (w/w).

Both concentrations were determined by HPLC

<u>Example 5.</u>

200 g of a mixture according to example 2 is provided in a container. 800 ml tap water is added to the container.

The mixture is stirred for 30 minutes with a stirrer (produced by HEIDOLPH) at room temperature.

At the end of the stirring procedure a non-clear liquid of white colour was obtained. The concentration of natamycin in the dispersion was 19.6 % by weight (w/w).

The concentration of LAE in the dispersion was 0.4% by weight (w/w).

Both concentrations were determined by HPLC

Example 6.

200 g of a mixture according to example 2 is provided in a container. 800 ml tap water is added to the container.

The mixture is stirred for 30 minutes with a stirrer (produced by HEIDOLPH) at room temperature.

At the end of the stirring procedure a non-clear liquid of white colour was obtained. The concentration of LAE in the dispersion was 10.0 % by weight (w/w).

The concentration of natamycin in the dispersion was 10.0 % by weight (w/w).

Both concentrations were determined by HPLC

Example 7.

An aqueous solution of natamycin is prepared by adding 1000 g of the dispersion of example 4 to 1000 g of tap water. The resulting liquid was stirred for 30 minutes.

The obtained solution was clear, with a very slight whitish colour.

The concentration of LAE in the solution was 19.8 % by weight (w/w).

The concentration of natamycin in the solution was 0.2 % (w/w).

The solution which was obtained was used for the treatment of fresh cheese.

Example 8.

An aqueous solution of natamycin is prepared by adding 10 g of the dispersion of example 5 to 970 g of tap water. The resulting liquid was stirred for 30 minutes.

The obtained solution was clear, with a very slight whitish colour.

The concentration of natamycin in the solution was 0.2 % by weight (w/w).

The concentration of LAE in the solution was 0.004 % (w/w).

The solution which was obtained was used for the treatment of cured cheese.

Example 9.

An aqueous solution of natamycin is prepared by adding 10 g of the dispersion of example 6 to 490 g of tap water. The resulting liquid was stirred for 30 minutes.

The obtained solution was clear.

The concentration of LAE in the solution was 0.2 % by weight (w/w).

The concentration of natamycin in the solution was 0.2 % (w/w).

The solution which was obtained was used for the treatment of semi-cured cheese.

Example 10.

Cured cheese was purchased from a local producer.

The cheese contains cow milk, sheep milk, goat milk, starters, salt, rennet. The minimum amount of fat 55% /dry weight. Minimum 45 dry weight.

Lauric arginate- from LAMIRSA with purity 90% Ethyl-N$^{\alpha}$-lauroyl-L-arginate HCl.

Natamycin- 95% in dry weight commercially available.

Inoculums is a mix of *Penicillum casicolum, Rizopus, Aspergillus niger* and *Cladosporium cladosporioides.* Spores seeded in Sabouraud Chloramphenirol agar are incubated at 25°C for 5 days. Spores are scraped of the agar with Ringer broth and diluted in Brain Heart Infusion broth. Brain Heart Infusion broth is incubated at 25°C for 5 days. Diluted inoculums are mixed at the desire concentration in order to have the mix inoculums.

In order to prepare the bath of natamycin with Lauric arginate, first the LAE was diluted in deionized water, once dissolved, natamycin was introduced and the liquid was left shaking during 30 minutes at least.

The cheese was sliced and cut in equal portions of 5 centimeters of diameter by 5 millimeters wide in sterile surroundings.

The portions of cheese were immersed in the bath for 5 seconds. They were dripped and dried for 5 minutes

in a sterile surface. After this, each sample was inoculated with *Penicillum casicolum, Rizopus, Aspergillus niger and Cladosporium cladosporioides* at target concentration of 4 log CFU/g.

**TREATMENTS**

Natamycin 95 % concentrations: two differently concentrated baths of natamycin were prepared: 500 ppm and 1000 ppm.

Lauric arginate concentrations: four differently concentrated baths of Lauric arginate were prepared: 0.5 %, 1 %, 5 % and 10 %.

Lauric Arginate with Natamycin 95 % concentrations: differently concentrated mixed baths of Lauric arginate and natamycin were prepared.

All treatments were compared with an untreated control and with each other.

1. Control.
2. 500 ppm natamycin
3. 1000 ppm natamycin
4. 0.5 % LAE
5. 1 % LAE
6. 5 % LAE
7. 10 % LAE
8. 500 ppm natamycin + 0.5 % LAE
9. 500 ppm natamycin + 5 % LAE

Storage temperature: samples were maintained at 22° C.

Analysis: measure the microbiocidal effect of Lauric arginate and natamycin treated samples against *Penicillum casicolum, Rizopus, Aspergillus niger* and *Cladosporium cladosporioides.* Analysis (Rose Bengal Agar, 25°C, 5 days) was carried out initially, at 5 days, at 10 days and at 15 days in triplicate. All microbiocidal effects were compared with the separate treatment with natamycin and Lauric arginate alone.

The first graph (figure 1) shows the inhibitory power of the separate treatment of Lauric arginate and natamycin, the best treatment is Lauric arginate at 10 %.

The second graph (figure 2) shows the synergy effects between Lauric arginate and natamycin, the mix of LAE 5% and natamycin 500 ppm is better than Lauric arginate 10%.

The third graph (figure 3) shows also the synergy effects between Lauric arginate and natamycin, the mix of Lauric arginate 0.5% and natamycin 500 ppm is similar than the effects of the natamycin 1000 ppm.

CONCLUSION

After analyzing the collected data of the study it was concluded that the treatment with Lauric arginate 10% is effective against the natural microorganisms cheese growth, but the mixture between the Lauric arginate 5% and the natamycin 500 ppm is the best with a less concentration of Lauric arginate, then the synergy is clear.

Example 11.

In this example the influence of the synergism of combinations of LAE and natamycin in the inventive composition has been investigated.

For that purpose propylene glycol solutions with different concentrations of LAE and natamyin were prepared.

LAE was produced by LAMIRSA, Terrassa; NATAMYCIN was purchased from SIGMA.

The effects of these preparations were investigated against the moulds *Aspergillus niger, Penicillium caseicolum, Cladosporium cladosporioides* and *Rhizopus,* the bacteria *Escherichia coli (ATCC* 8739), *Salmonella typhimurium* (ATCC 14028), *Listeria monocytogenes* (ATCC 15313), *Bacillus subtilis* (ATCC 6633) and the yeasts *Candida albicans* (ATCC 10231) and *Saccharomyces cerevisiae* (ATCC 9763).

In the next tables 1 to 10 the effect of LAE and natamycin is displayed when administered alone and in combination. The table indicates the MIC values found at different relationship between natamycin and LAE, whereby a value for natamycin : LAE 1:1 means the same concentration for both substances in the antimicrobial composition.

The interaction of the two components of the antimicrobial mixture is calculated according to the method described by Kull et al. (Kull F.C., Eisman P.C., Sylwestrowicz H.D. and Mayer R.L., Applied Microbiology, 1961; 6: 538-541). According to this method the so-called synergy index is calculated according to the following formula:

$$\text{Synergy index SI} = Q_{lae}/Q_{LAE} + Q_n/Q_N.$$

The elements used for the calculation of the synergy index according to the above formula have the following meaning:

$Q_{lae}$ = minimum inhibition concentration of LAE in the mixture of LAE and natamycin,
$Q_{LAE}$ = minimum inhibition concentration of LAE as single antimicrobial without natamycin,
$Q_n$ = minimum inhibition concentration of natamycin in the mixture of LAE and natamycin and
$Q_N$ = minimum inhibition concentration of natamycin as single antimicrobial without LAE.

All indicated symbols indicate a particular concentration leading to a particular end point, in this case the inhibition of growth of the microorganisms, so that the selected end point is in fact the minimal inhibitory concentration (MIC).

The method of Kull et al. for the calculation of the synergy index allows a very quick evaluation of the type of interaction displayed by the two components of the antimicrobial mixture. When the synergy index displays a value of more than 1, then there is an antagonism between the two components. When the synergy index is 1, then there is an addition of the effects of the two components. When the synergy index displays a value of less than 1, then there is a synergism between the two components.

**<u>Synergy values from the solution of LAE and natamycin</u>**

<u>Terminology:</u>

$Q_n$: MIC of natamycin in the mixture of natamycin and LAE
$Q_N$:MIC of natamycin alone
$Q_{lae}$: MIC of LAE in the mixture of natamycin and LAE
$Q_{LAE}$: MIC of LAE alone
SI: Synergy Index

The MIC values of natamycin alone ($Q_N$) reported in tables 5 to 8 corresponds to the maximum value studied. However, the real MIC of natamycin is higher than the maximum values studied (i.e.: 1024, 2048), accordingly the real SI should be lower than the SI reported in the tables.

**Table 1.**

***Aspergillus niger***

| Relationship Natamycin :LAE | $Q_n$ | $Q_{lae}$ | $Q_N$ | $O_n/O_N$ | $Q_{LAE}$ | $Q_{lae}/Q_{LAE}$ | **SI** |
|---|---|---|---|---|---|---|---|
| 1:1 | 1 | 1 | 2 | 0,5 | 128 | 0,0078 | 0,508 |
| 1:5 | 1 | 5 | 2 | 0,5 | 128 | 0,0391 | 0,539 |
| 1:10 | 1 | 10 | 2 | 0,5 | 128 | 0,0781 | 0,578 |
| 1:100 | 0,5 | 50 | 2 | 0,25 | 128 | 0,3906 | 0,641 |
| 1:500 | 0,1 | 50 | 2 | 0,05 | 128 | 0,3906 | 0,441 |

**Table 2.**

***Penicillium caseicolum***

| Relationship Natamycin:LAE | $Q_n$ | $Q_{lae}$ | $Q_N$ | $O_n/O_N$ | $Q_{LAE}$ | $Q_{lae}/Q_{LAE}$ | **SI** |
|---|---|---|---|---|---|---|---|
| 1:1 | 0,5 | 0,5 | 1 | 0,5 | 256 | 0,0020 | 0,502 |
| 1:5 | 0,5 | 2,5 | 1 | 0,5 | 256 | 0,0098 | 0,510 |
| 1:10 | 0,5 | 5 | 1 | 0,5 | 256 | 0,0195 | 0,520 |
| 1:100 | 0,5 | 50 | 1 | 0,5 | 256 | 0,1953 | 0,695 |
| 1:500 | 0,25 | 125 | 1 | 0,25 | 256 | 0,4883 | 0,738 |

**Table 3.**

***Cladosporium cladosporioides***

| Relationship natamycin:LAE | $Q_n$ | $Q_{lae}$ | $Q_N$ | $O_n/O_N$ | $Q_{LAE}$ | $Q_{lae}/Q_{LAE}$ | **SI** |
|---|---|---|---|---|---|---|---|
| 1:1 | 0,5 | 0,5 | 1 | 0,5 | 64 | 0,0078 | 0,508 |
| 1:5 | 0,5 | 2,5 | 1 | 0,5 | 64 | 0,0391 | 0,539 |
| 1:10 | 0,5 | 2,5 | 1 | 0,5 | 64 | 0,0391 | 0,539 |
| 1:100 | 0,25 | 25 | 1 | 0,25 | 64 | 0,3906 | 0,641 |
| 1:500 | 0,1 | 50 | 1 | 0,1 | 64 | 0,7813 | 0,881 |

**Table 4.**

***Rhizopus***

| Relationship Natamycin:LAE | $Q_n$ | $Q_{lae}$ | $Q_N$ | $O_n/O_N$ | $Q_{LAE}$ | $Q_{lae}/Q_{LAE}$ | **SI** |
|---|---|---|---|---|---|---|---|
| 1:1 | 3 | 3 | 4 | 0,75 | 648 | 0,0046 | 0,755 |
| 1:5 | 3 | 12 | 4 | 0,75 | 648 | 0,0185 | 0,769 |
| 1:10 | 3 | 30 | 4 | 0,75 | 648 | 0,0463 | 0,796 |
| 1:100 | 2 | 200 | 4 | 0,50 | 648 | 0,3086 | 0,809 |
| 1:500 | 0,5 | 250 | 4 | 0,13 | 648 | 0,3858 | 0,511 |

**Table 5.**

***Escherichia coli* ATCC 8739**

| Relationship Natamycin:LAE | $Q_n$ | $Q_{lae}$ | $Q_N$ | $O_n/O_N$ | $Q_{LAE}$ | $Q_{lae}/Q_{LAE}$ | **SI** |
|---|---|---|---|---|---|---|---|
| 1:1 | 22 | 22 | 1024 | 2,15E-02 | 32 | 0,6875 | 0,709 |
| 1:5 | 5 | 25 | 1024 | 4,88E-03 | 32 | 0,7813 | 0,786 |
| 1:10 | 2 | 20 | 1024 | 1,95E-03 | 32 | 0,6250 | 0,627 |
| 1:100 | 0,25 | 25 | 1024 | 2,44E-04 | 32 | 0,7813 | 0,781 |
| 1:500 | 0,05 | 25 | 1024 | 4,88E-05 | 32 | 0,7813 | 0,781 |

**Table 6.**

***Salmonella typhimurium ATCC 14028***

| Relationship Natamycin:LAE | $Q_n$ | $Q_{lae}$ | $Q_N$ | $O_n/O_N$ | $Q_{LAE}$ | $Q_{lae}/Q_{LAE}$ | **SI** |
|---|---|---|---|---|---|---|---|
| 1:1 | 21 | 21 | 2048 | 1,03E-02 | 32 | 0,6563 | 0,667 |
| 1:5 | 5 | 25 | 2048 | 2,44E-03 | 32 | 0,7813 | 0,784 |
| 1:10 | 2 | 20 | 2048 | 9,77E-04 | 32 | 0,6250 | 0,626 |
| 1:100 | 0,25 | 25 | 2048 | 1,22E-04 | 32 | 0,7813 | 0,781 |
| 1:500 | 0,05 | 25 | 2048 | 2,44E-05 | 32 | 0,7813 | 0,781 |

**Table 7.**

***Listeria monocytogenes ATCC 15313***

| Relationship Natamycin:LAE | $Q_n$ | $Q_{lae}$ | $Q_N$ | $O_n/O_N$ | $Q_{LAE}$ | $Q_{lae}/Q_{LAE}$ | **SI** |
|---|---|---|---|---|---|---|---|
| 1:1 | 20 | 20 | 1024 | 1,95E-02 | 32 | 0,6250 | 0,645 |
| 1:5 | 5 | 25 | 1024 | 4,88E-03 | 32 | 0,7813 | 0,786 |
| 1:10 | 2 | 20 | 1024 | 1,95E-03 | 32 | 0,6250 | 0,627 |
| 1:100 | 0,25 | 25 | 1024 | 2,44E-04 | 32 | 0,7813 | 0,781 |
| 1:500 | 0,05 | 25 | 1024 | 4,88E-05 | 32 | 0,7813 | 0,781 |

**Table 8.**

***Bacillus subtilis ATCC 6633***

| Relationship Natamycin:LAE | $Q_n$ | $Q_{lae}$ | $Q_N$ | $O_n/O_N$ | $Q_{LAE}$ | $Q_{lae}/Q_{LAE}$ | **SI** |
|---|---|---|---|---|---|---|---|
| 1:1 | 10 | 10 | 2048 | 4,88E-03 | 16 | 0,6250 | 0,630 |
| | | | | | | | |
| 1:1 | 10 | 10 | 2048 | 4,88E-03 | 16 | 0,6250 | 0,630 |
| 1:5 | 2 | 10 | 2048 | 9,77E-04 | 16 | 0,6250 | 0,626 |
| 1:10 | 1 | 10 | 2048 | 4,88E-04 | 16 | 0,6250 | 0,625 |
| 1:100 | 0,1 | 10 | 2048 | 4,88E-05 | 16 | 0,6250 | 0,625 |
| 1:500 | 0,02 | 10 | 2048 | 9,77E-06 | 16 | 0,6250 | 0,625 |

**Table 9.**

***Candida albicans ATCC 10231***

| Relationship Natamycin:LAE | $Q_n$ | $Q_{lae}$ | $Q_N$ | $Q_n/Q_N$ | $Q_{LAE}$ | $Q_{lae}/Q_{LAE}$ | **SI** |
|---|---|---|---|---|---|---|---|
| 1:1 | 1 | 1 | 2 | 5,00E-01 | 16 | 0,0625 | 0,563 |
| 1:5 | 0,7 | 3,5 | 2 | 3,50E-01 | 16 | 0,2188 | 0,569 |
| 1:10 | 0,5 | 5 | 2 | 2,50E-01 | 16 | 0,3125 | 0,563 |
| 1:100 | 0,1 | 10 | 2 | 5,00E-02 | 16 | 0,6250 | 0,675 |
| 1:500 | 0,02 | 10 | 2 | 1,00E-02 | 16 | 0,6250 | 0,635 |

**Table 10.**

***Saccharomyces cerevisiae ATCC 9763***

| Relationship Natamycin:LAE | $Q_n$ | $Q_{lae}$ | $Q_N$ | $Q_n/Q_N$ | $Q_{LAE}$ | $Q_{lae}/Q_{LAE}$ | **SI** |
|---|---|---|---|---|---|---|---|
| 1:1 | 2 | 2 | 2,5 | 8,00E-01 | 32 | 0,0625 | 0,863 |
| 1:5 | 1,5 | 7,5 | 2,5 | 6,00E-01 | 32 | 0,2344 | 0,834 |
| 1:10 | 1 | 10 | 2,5 | 4,00E-01 | 32 | 0,3125 | 0,713 |
| 1:100 | 0,25 | 25 | 2,5 | 1,00E-01 | 32 | 0,7813 | 0,881 |
| 1:500 | 0,05 | 25 | 2,5 | 2,00E-02 | 32 | 0,7813 | 0,801 |

## Claims

**1.** A solid composition consisting of natamycin and a cationic surfactant (LAE) of the following formula

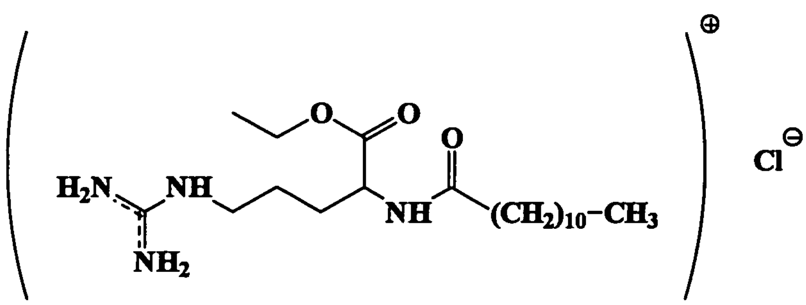

(2)

the solid composition consisting of 2-99.9 % by weight of LAE and 0.1-98 % by weight of natamycin, the sum being 100%,

2. The solid composition according to claim 1, furthermore comprising components such as sugar, salt, anticakings, antioxidants, chelating agents, or thickening agents,
in an amount of 0-5 parts by weight per 100 parts by weight of the amount of LAE and natamycin.

3. A method for preparing a dispersion of natamycin by the dispersion of the solid composition according to claim 1 or 2 in a solvent.

4. The method of claim 3, in which the solvent is tap water or an organic solvent such as propylene glycol or glycerine, or a further food-grade solvent, or a combination of these solvents.

5. A dispersion of natamycin prepared by the method of claim 3 or 4, wherein the concentration of natamycin is between 100 ppm and 10,000 ppm, optionally containing further components such as sugar, salt, anticakings, antioxidants, chelating agents, surfactants or thickening agents.

6. A method for preparing an aqueous solution of natamycin by diluting the dispersion of claim 5 with water.

7. A method for preparing an aqueous solution of natamycin by dissolving the solid composition of claim 1 or 2 in water.

8. Aqueous solution obtainable by the method of claim 6 or 7, containing natamycin in a concentration of 10 to 10,000 ppm in combination with LAE in an amount of 200 to 100,000 ppm and optionally comprising further components such as sugar, salt, anticakings, antioxidants, chelating agents, surfactants or thickening agents.

9. The aqueous solution according to claim 8, in which the ratio by weight of natamycin to LAE is 1:20 to 1:200.

10. The aqueous solution according to claim 8 or 9, in which the preferred ratio by weight of natamycin to LAE is 1:50 to 1:100.

11. The aqueous solution according to any one of claims 8 to 10, wherein the concentration of natamycin is 0.001 to 0.15 % (w/w).

12. Use of the aqueous solution of any one of claims 8 to 11 for the treatment of food products.

13. The use according to claim 12 for the treatment of cheese, cold meat products and bakery products.

14. The use according to claim 12 for the treatment of liquid preparations such as drinking fluids.

15. The use of the preparations of any of the claims 1, 2, 5 and 8 to 11 for the treatment of cosmetic preparations.

16. The use of the preparations of any of the claims 1, 2, 5, and 8 to 11 for the preparation of medicaments to treat infections in animals or human beings.

**Patentansprüche**

1. Feste Zusammensetzung, bestehend aus Natamycin und einem kationischen oberflächenaktiven Stoff (LAE) der folgenden Formel

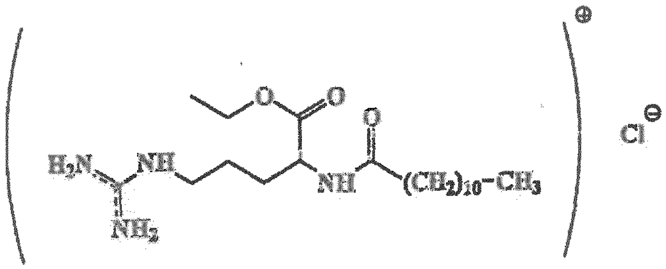

(2)

wobei die feste Zusammensetzung aus 2 bis 99,9 Gew.-% LAE und 0,1 bis 98 Gew.-% Natamycin besteht, wobei die Summe 100 % beträgt.

2. Feste Zusammensetzung nach Anspruch 1, darüber hinaus umfassend Komponenten wie Zucker, Salz, Antiverbackungsmittel, Antioxidantien, Gelatisierungsmittel oder Verdickungsmittel,
in einer Menge von 0 bis 5 Gewichtsteilen pro 100 Gewichtsteilen der Menge an LAE und Natamycin.

3. Verfahren zur Herstellung einer Dispersion von Natamycin durch Dispergieren der festen Zusammensetzung nach Anspruch 1 oder 2 in einem Lösungsmittel.

4. Verfahren nach Anspruch 3, bei dem das Lösungsmittel Leitungswasser oder ein organisches Lösungsmittel wie Propylenglykol oder Glycerin ist, oder ein weiteres Lebensmittelgrad-Lösungsmittel oder eine Kombination dieser Lösungsmittel.

5. Dispersion von Natamycin, hergestellt durch das Verfahren nach Anspruch 3 oder 4, wobei die Konzentration an Natamycin zwischen 100 ppm und 10.000 ppm liegt, gegebenenfalls enthaltend weitere Komponenten wie Zucker, Salz, Antiverbackungsmittel, Antioxidantien, Gelatisierungsmittel, oberflächenaktive Stoffe oder Verdickungsmittel.

6. Verfahren zur Herstellung einer wässrigen Lösung von Natamycin durch Verdünnen der Dispersion aus Anspruch 5 mit Wasser.

7. Verfahren zur Herstellung einer wässrigen Lösung von Natamycin durch Lösen der festen Zusammensetzung nach Anspruch 1 oder 2 in Wasser.

8. Wässrige Lösung, erhältlich durch das Verfahren nach Anspruch 6 oder 7, enthaltend Natamycin in einer Konzentration von 10 bis 10.000 ppm in Kombination mit LAE in einer Menge von 200 bis 100.000 ppm, und gegebenenfalls umfassend weitere Komponenten wie Zucker, Salz, Antiverbackungsmittel, Antioxidantien, Gelatisierungsmittel, oberflächenaktive Stoffe oder Verdickungsmittel.

9. Wässrige Lösung nach Anspruch 8, wobei das Gewichtsverhältnis von Natamycin zu LAE 1 : 20 bis 1 : 200 beträgt.

10. Wässrige Lösung nach Anspruch 8 oder 9, wobei das bevorzugte Gewichtsverhältnis von Natamycin zu LAE 1 : 50 bis 1 : 100 beträgt.

11. Wässrige Lösung nach einem der Ansprüche 8 bis 10, wobei die Konzentration von Natamycin 0,001 bis 0,15 % (m/m) beträgt.

12. Verwendung der wässrigen Lösung nach einem der Ansprüche 8 bis 11 zur Behandlung von Lebensmittelprodukten.

13. Verwendung nach Anspruch 12 für die Behandlung von Käse, kalten Fleischprodukten und Bäckereiprodukten.

14. Verwendung nach Anspruch 12 für die Behandlung von flüssigen Präparaten wie Trinkflüssigkeiten.

15. Verwendung der Präparate nach einem der Ansprüche 1, 2, 5 und 8 bis 11 zur Behandlung von kosmetischen Präparaten.

16. Verwendung der Präparate nach einem der Ansprüche 1, 2, 5 und 8 bis 11 zur Herstellung von Medikamenten zur Behandlung von Infektionen bei Tieren oder Menschen.

## Revendications

1. Composition solide consistant en la natamycin et un agent tensioactif cationique (LAE) de la formule suivante

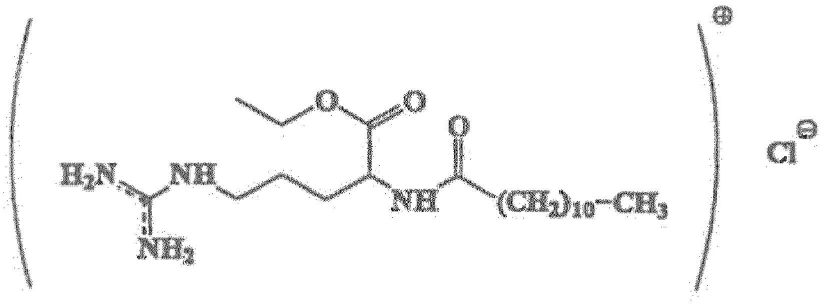

(2)

la composition solide consistant en 2-99,9 % en poids de LAE et 0,1-98 % en poids de natamycin, la somme étant 100 %.

2. Composition solide selon la revendication 1, comprenant en outre des composants tels que le sucre, le sel, les antiagglomérants, les antioxydants, les chélateurs ou les épaisseurs,
dans une quantité de 0-5 parts par poids sur 100 parts par poids de la quantité de LAE et de la natamycin.

3. Procédé pour la préparation d'une dispersion de natamycin par la dispersion de la composition solide selon la revendication 1 ou 2 dans un solvant.

4. Procédé selon la revendication 3, dans lequel le solvant est l'eau du robinet ou un solvant organique tel que le polypropylène glycol ou le glycérine, ou un autre solvant de qualité alimentaire, ou une association de ces solvants.

5. Dispersion de natamycin préparée par le procédé selon la revendication 3 ou 4, dans laquelle la concentration de natamycin est entre 100 ppm et 10.000 ppm, le cas échéant comprenant d'autres composants tel que le sucre, le sel, les antiagglomérants, les antioxydants, les chélateurs ou les épaisseurs.

6. Procédé pour la préparation d'une solution aqueuse de natamycin en diluant la dispersion selon la revendication 5 avec de l'eau.

7. Procédé pour la préparation d'une solution aqueuse de natamycin en dissolvant la composition solide selon la revendication 1 ou 2 dans l'eau.

8. Solution aqueuse qui est accessible par le procédé selon la revendication 6 ou 7, contenant la natamycin dans une concentration de 10 à 10.000 ppm en association avec LAE dans une quantité de 200 à 100.000 ppm et le cas échéant comprenant d'autres composants tel que le sucre, le sel, les antiagglomérants, les antioxydants, les chélateurs ou les épaisseurs.

9. Solution aqueuse selon la revendication 8, dans laquelle le rapport par poids de natamycin à LAE est 1:20 à 1:200.

10. Solution aqueuse selon la revendication 8 ou 9, dans laquelle le rapport par poids préféré de natamycin à LAE est 1:50 à 1:100.

11. Solution aqueuse selon l'une des revendications 8 à 10, dans laquelle la concentration de natamycin est 0,001 à 0,15 % (poids par rapport au poids).

12. Utilisation de la solution aqueuse selon l'une des revendications 8 à 11 pour le traitement des produits alimentaires.

13. Utilisation selon la revendication 12 pour le traitement du fromage, des produits de viande froids et des produits de boulangerie.

14. Utilisation selon la revendication 12 pour le traitement des préparations liquides tel que des fluides potables.

15. Utilisation des préparations selon l'une des revendications 1, 2, 5 et 8 à 11 pour le traitement des préparations cosmétiques.

**16.** Utilisation des préparations selon l'une des revendications 1, 2, 5 et 8 à 11 pour la préparation des médicaments afin de traiter des infections dans des animaux ou des êtres humaines.

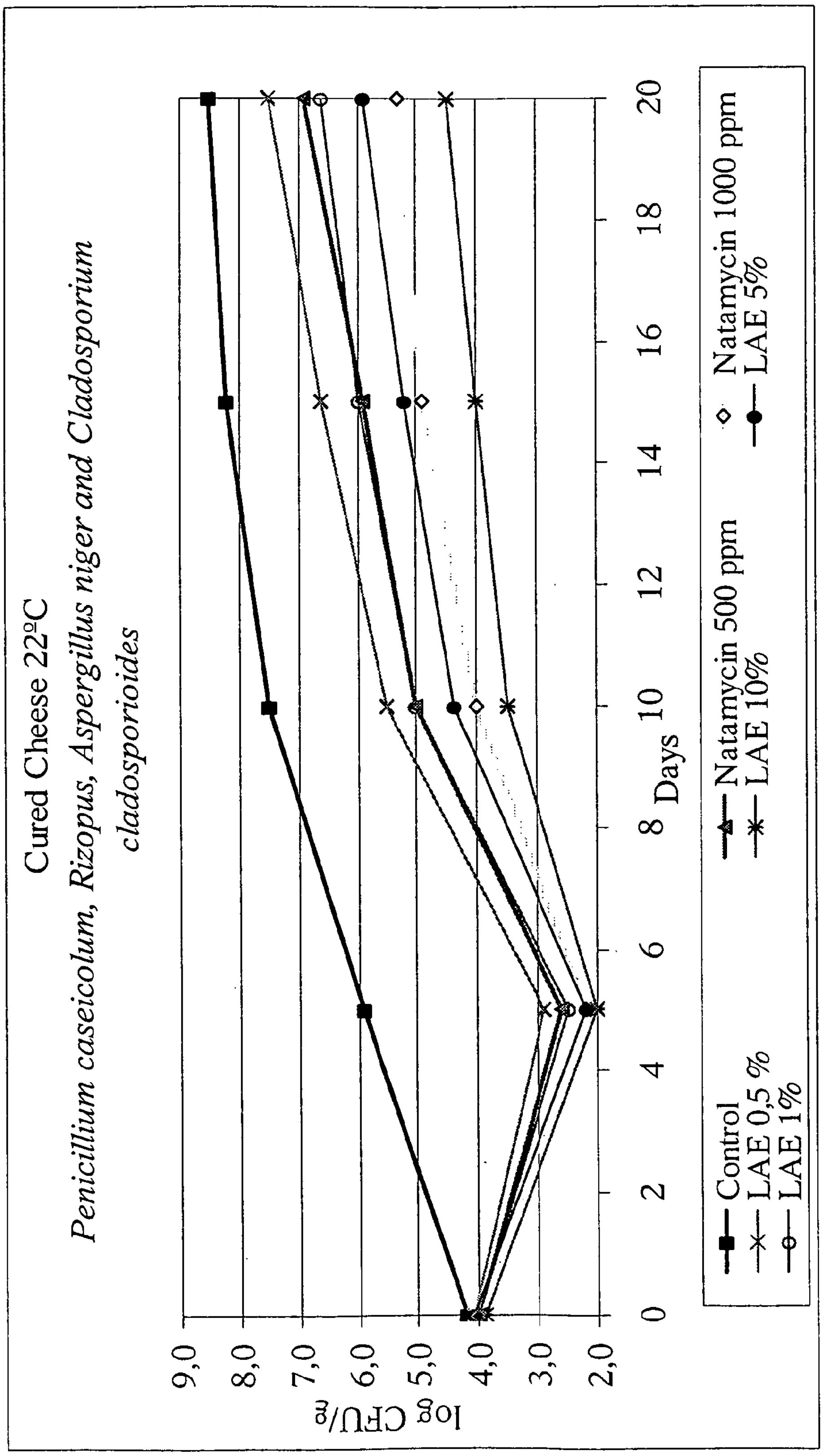
Cured Cheese 22ºC
Penicillium caseicolum, Rizopus, Aspergillus niger and Cladosporium cladosporioides
log CFU/g
9,0
8,0
7,0
6,0
5,0
4,0
3,0
2,0
0
2
4
6
8
10
12
14
16
18
20
Days
Control
LAE 0,5 %
LAE 1%
Natamycin 500 ppm
LAE 10%
Natamycin 1000 ppm
LAE 5%

Figure 1.

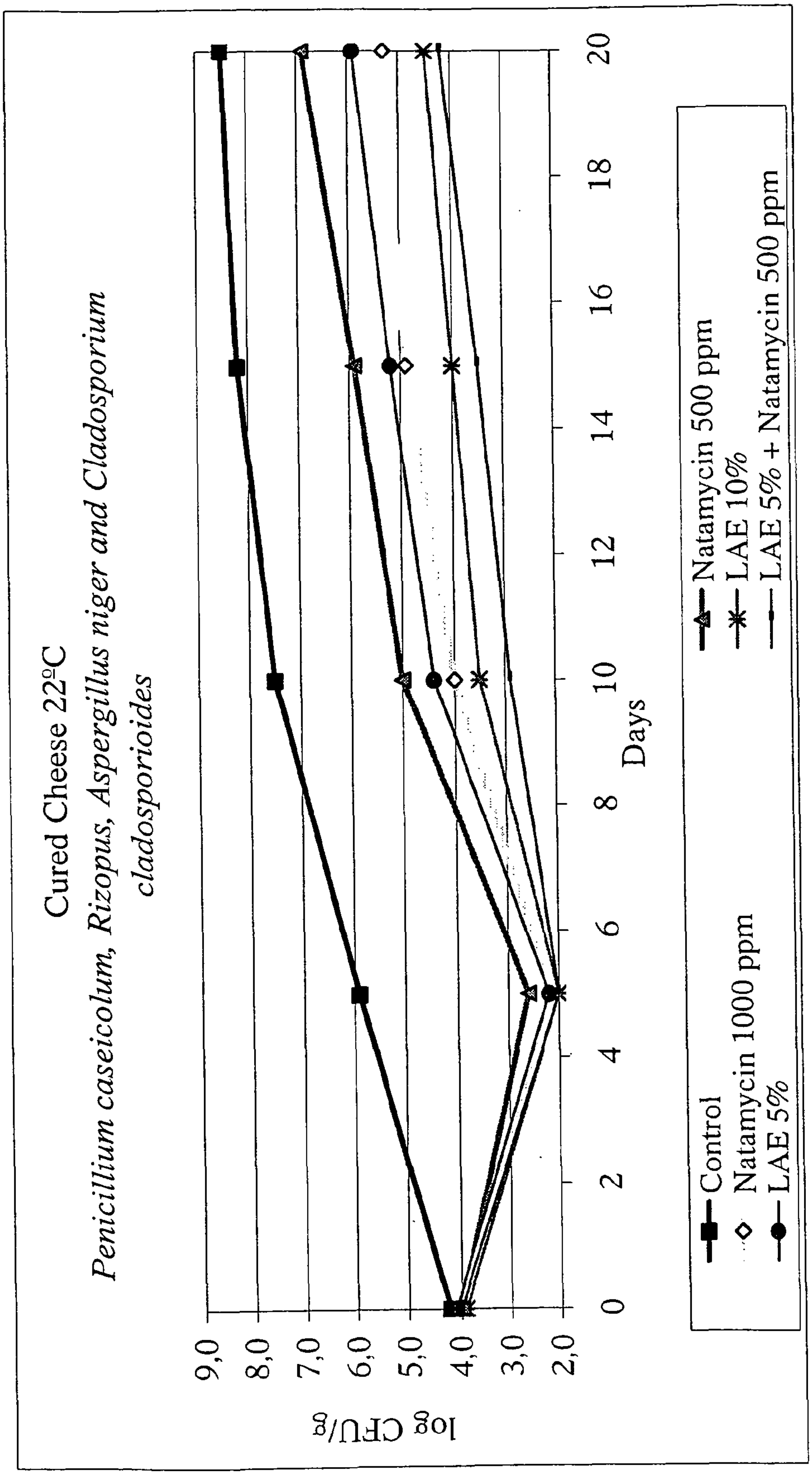
Cured Cheese 22ºC
Penicillium caseicolum, Rizopus, Aspergillus niger and Cladosporium cladosporioides
log CFU/g
9,0
8,0
7,0
6,0
5,0
4,0
3,0
2,0
0
2
4
6
8
10
12
14
16
18
20
Days
Control
Natamycin 1000 ppm
LAE 5%
Natamycin 500 ppm
LAE 10%
LAE 5% + Natamycin 500 ppm

Figure 2.

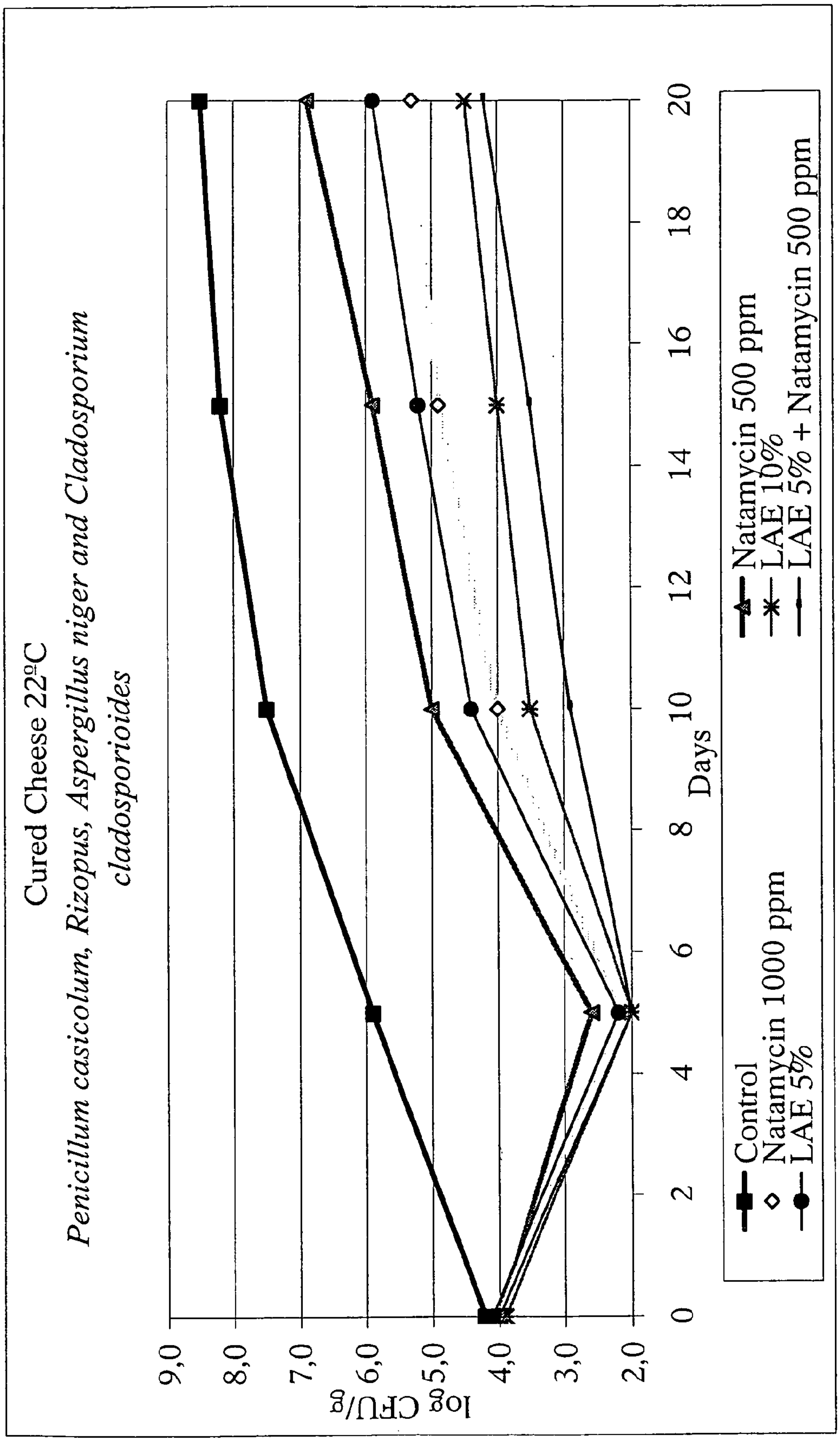
Cured Cheese 22ºC
Penicillum casicolum, Rizopus, Aspergillus niger and Cladosporium cladosporioides
log CFU/g
9,0
8,0
7,0
6,0
5,0
4,0
3,0
2,0
0
2
4
6
8
10
12
14
16
18
20
Days
Control
Natamycin 1000 ppm
LAE 5%
Natamycin 500 ppm
LAE 10%
LAE 5% + Natamycin 500 ppm

Figure 3.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0678241 B1
- EP 0670676 B1
- US 5597598 A
- US 6146675 A
- WO 2005097063 A
- WO 03034842 A
- WO 03013453 A
- WO 03013454 A
- WO 03043593 A
- ES 512643
- WO 9621642 A
- WO 0194292 A
- WO 03064669 A
- WO 03094638 A
- US 7074447 B
- EP 1450608 B1
- WO 2006084553 A1
- WO 2004082407 A1
- WO 2007112379 A1

### Non-patent literature cited in the description

- **KULL F.C. ; EISMAN P.C. ; SYLWESTROWICZ H.D. ; MAYER R.L.** *Applied Microbiology,* 1961, vol. 6, 538-541